Europäisches Patentamt

European Patent Office

Office européen des brevets

(11) Publication number: **0 118 908**
**A2**

(12) **EUROPEAN PATENT APPLICATION**

(21) Application number: 84102620.6

(22) Date of filing: 09.03.84

(51) Int. Cl.³: **C 12 P 21/00**
**A 61 K 37/02**

(30) Priority: 11.03.83 JP 39146/83

(43) Date of publication of application:
19.09.84 Bulletin 84/38

(84) Designated Contracting States:
AT BE CH DE FR GB IT LI NL SE

(71) Applicant: Chugai Seiyaku Kabushiki Kaisha
5-1, 5-chome, Ukima Kita-ku
Tokyo(JP)

(71) Applicant: Kumatori, Toshiyuki
9-1, Anagawa 4-chome
Chiba-shi Chiba-ken(JP)

(72) Inventor: Ono, Masayoshi
1369-7, Yamaguchi
Tokorozawa-shi Saitama-ken(JP)

(72) Inventor: Hirashima, Kunitake
5-19-9, Nakano
Nakano-ku Tokyo(JP)

(72) Inventor: Bessho, Masami
1-19-6-202, Kameido
Koto-ku Tokyo(JP)

(72) Inventor: Imazeki, Ikuo
3-36-13, Nakamura
Nerima-ku Tokyo(JP)

(72) Inventor: Nomura, Hitoshi c/o Chugai Seiyaku
Nakamuraryo of 2-4-8-306, Azusawa
Itabashi-ku Tokyo(JP)

(74) Representative: Vossius Vossius Tauchner Heunemann
Rauh
Siebertstrasse 4 P.O. Box 86 07 67
D-8000 München 86(DE)

(54) CSF inhibitory substance.

(57) A substance inhibiting colony stimulating factor (CSF) which is accumulated in a culture medium during cultivation of CSF-producing cells and has physicochemical characteristics defined in the specification. This substance exhibits an inhibitory action against CSF and is also expected to be useful as an antileukemic agent.

Croydon Printing Company Ltd.

CHUGAI SEUYAKU K.K. and
Toshiyuki KUMATORI
Our Ref: S 904 EP

— 1 —

VOSSIUS · VOSSIUS · TAUCHNER
· HEUNEMANN · RAUH
PATENTANWÄLTE
SIEBERTSTR. 4, 8000 MÜNCHEN 80
TEL (089) 47 40 75

0118908

9. März 1984

## CSF INHIBITORY SUBSTANCE

This invention relates to a substance inhibiting colony stimulating factor which is referred to as "CSF inhibitory substance" hereunder.

The inventors of this invention found that on culturing a T3M-5 cell line (CSF-producing cells which had been established from human thyroid gland cancer cells) at a high concentration the CSF activity was not proportional to its concentration, but was weaker than the expected level. They continued their study to seek the cause of the phenomenon and found the presence of a CSF inhibitory substance in the solution and isolated it to complete this invention.

The CSF inhibitory substance isolated according to this invention has the following physicochemical characteristics.

1) Molecular Weight:

50,000 - 60,000 by gel filtration using Sephadex G-75 (Pharmacia Fine Chemicals),

| | |
|---|---|
| column size: | 10 mm diameter, 1,000 mm height, |
| bed volume: | 78.5 ml, |
| flow rate: | 4.0 ml/hr, and |
| eluent: | 0.01 M Tris-HCl buffer solution containing 0.15 M NaCl (pH 7.4). |

The standard curve was drawn on the basis of the volume of eluate with respect to the following proteins, and the molecular weight of the CSF inhibitory substance was determined based on the standard curve.

| protein | molecular weight |
|---|---|
| γ-globulin | 160,000 |
| albumin | 66,000 |
| ovalbumin | 45,000 |
| chymotrypsinogen A | 25,700 |
| cytochrome C | 12,400 |

2) Isoelectric Point: pH 4.7

The isoelectric point was determined by the flat bed isoelectric focusing method using Sephadex IEF (Pharmacia Fine Chemicals). The pH gradient in the bed from 2.5 to 5.0 was performed by electrophoresis with use of pharmalyte (Pharmacia Fine Chemicals) at 8 watts for 45 minutes, and after applying the CSF inhibitory substance (144 mg) to the bed, electrophoresed at 60 watts for 5 hours to determine the isoelectric point.

3) pH-Stability:

Stable at a pH of from 5 to 9. Inactivated at a pH lower than 3 or higher than 11.

The CSF inhibitory substance was added to the buffer solutions of various pH, allowed to stand for 24 hours, and dialyzed against 0.01 M Tris-HCl buffer solution (pH 7) for one day, and then the CSF inhibitory activity was determined.

4) Absorption in U.V. range:

Maximum absorption at 280 nm

5) Reactions with Enzymes:

Not inactivated with DNase (3.175 IU), RNase (76 IU) or trypsin (0.2%);

Inactivated completely with pronase (5 IU: Kaken Chemical Co., Ltd., Japan).

6) Thermostability:

Stable at 37°C and almost completely inactivated at 56°C for 30 min.

These characteristics of the substance of this invention differ in distribution, molecular weight, isoelectric point, etc., from those of several known substances inhibiting CSF, and this shows that the substance of this invention is novel.

If fetal calf serum (FCS) which will be added to the culture medium used in the Example below is treated as in the Example, no substance of this invention is detected.

This proves that the substance of this invention is not derived from serum.

In addition to the above explained characteristics, the CSF inhibiting substance exhibits an activity which inhibits the colony formation of a K-562 cell line of human leukemic cells which is able to maintain their multiplication independent of the presence of CSF. This activity is observed at such a remarkably low concentration that normal cells are not adversely affected. Therefore, the substance is expected to be useful as an antileukemic agent.

Example

T3M-5 cells established from human thyroid gland cancer cells as CSF-producing cells were suspended in a 10% FCS-containing F-10 medium (500 ml) in a count of 1.0-2.0 x $10^7$. This cell line is preserved at Third Department of Internal Medicine, Faculty of Medicine, University of Tokyo; The Central Institute for Experimental Animals; and The Laboratories of Chugai Seiyaku Kabushiki Kaisha. The suspension was cultured in a roller bottle at 0.5 rpm. at 37°C for one week.

The supernatants of broth cultures similarly cultured in 20 bottles were combined to give a total amount of about 10ℓ. After concentration thereof, the combined supernatants was subjected to a gel filtration with Sephadex G-75 which had been equilibrated with 0.01 M Tris-HCl buffer solution containing 0.15 M NaCl (pH 7.4) and 0.01% Tween 20 and fractions containing a substance with 50,000 - 60,000 molecular weight were collected.

The fractions were applied to and absorbed on DEAE-Sepharose CL-6B, and eluted with a buffer solution stepwisely increasing the concentration of NaCl, i.e. 0.04 M, 0.08 M and 0.15 M. Then, the eluate with a 0.1 M citric acid buffer solution containing 0.5 M NaCl were collected as CSF inhibiting substance containing fractions.

After concentration with PM-10 the volume of the fractions containing CSF inhibiting substance was 7 ml which contained 1,000 mg of protein.

In the colony assay method using mouse bone marrow derived cells in the presence of a sufficient amount of CSF, the addition of about 1,200 μg of the protein exhibited 90% inhibition. In contrast, 90% inhibition was observed at about 120 μg of the protein when human bone marrow derived cells were cultivated.

Claims:

1.    A CSF inhibitory substance which is accumulated in a culture medium of a CSF-producing cell line and has the following physicochemical characteristics:

Molecular Weight:

50,000 - 60,000 by gel filtration,

Isoelectric Point:

pH 4.7,

pH-Stability:

Stable at a pH of from 5 - 9, and inactivated at a pH lower than 3 or higher than 11,

U.V.-Absorption:

Maximum absorption at 280 nm,

Stability against Enzymes:

Not inactivated with DNase (3.175 IU), RNase (76 IU) or trypsin (0.2%), and inactivated completely with pronase (5 IU; Kaken Chemical Co., Ltd.), and

Thermostability:

Stable at 37°C, and almost completely inactivated at 56°C for 30 min.

2.    CSF inhibitory substance according to claim 1, wherein the CSF-producing cell line is a T3M-5 cell line established from human thyroid gland cancer cells.

3.    Pharmaceutical composition for use in the inhibition of undesired cell multiplication comprising the CSF inhibitory substance according to claim 1 or 2 and usual carriers, adjuvants or diluants.

4.    The CSF inhibitory substance according to claims 1 or 2 for use in the inhibition of undesired cell multiplication.